# FASCICULE DE BREVET EUROPEEN

(11) **EP 1 499 266 B1**
(45) Date de publication et mention de la délivrance du brevet: **19.02.2014**
(21) Numéro de dépôt: 03740651.9
(22) Date de dépôt: 22.04.2003
(51) Int. Cl.: A61L 27/16, A61L 27/26, A61F 2/24

(54) **PROCEDE DE REALISATION D UNE PROTHESE DE VALVE CARDIAQUE AOR TIQUE OU MITRALE ET PROTHESE DE VALVE CARDIAQUE AORTIQUE OU MITRALE AINSI OBTENUE**
VERFAHREN ZUR HERSTELLUNG EINER HERZKLAPPENPROTHESE UND DAMIT HERGESTELLTE AORTENKLAPPENPROTHESE ODER MITRALKLAPPENPROTHESE
METHOD FOR PRODUCING AN AORTIC OR MITRAL HEART VALVE PROSTHESIS AND RESULTING AORTIC OR MITRAL HEART VALVE

(30) Priorité: 23.04.2002 FR 0205086; 27.09.2002 FR 0212030
(43) Date de publication de la demande: 26.01.2005
(73) Titulaire: Université de Haute Alsace, 68093 Mulhouse cedex (FR); Université de Strasbourg, 67000 Strasbourg (FR); Hôpitaux Universitaires de Strasbourg, 67091 Strasbourg cedex (FR)
(72) Inventeur: HEIM, Frédéric, F-68420 Hattstatt (FR); DURAND, Bernard, F-68120 Pfastatt (FR); KRETZ, Jean-Georges, F-67100 Strasbourg (FR); CHAKFE, Nabil, F-67114 Eschau (FR)
(74) Mandataire: Ziegler, Stefan Michael
(86) Numéro de dépôt international: PCT/FR2003/001271
(87) Numéro de publication internationale: WO 2003/090645

(56) Documents cités:
- EP-A- 0 331 345
- WO-A-01/52776
- CH-A- 510 433
- FR-A- 2 728 457
- US-A- 2 832 078
- US-A- 5 500 015
- US-A- 5 713 953

## Description

La présente invention concerne le domaine de la médecine, en particulier de la chirurgie réparatrice, et notamment de la chirurgie cardiaque, plus particulièrement des prothèses cardiaques, et a pour objet un procédé de réalisation d'une prothèse de valve cardiaque aortique ou mitrale.

L'invention a également pour objet une prothèse de valve cardiaque aortique ou mitrale obtenue par mise en oeuvre de ce procédé.

L'implantation de prothèses de valve cardiaque aortique est généralement nécessaire pour pallier des insuffisances dues à la dégénérescence de ladite valve, ce plus particulièrement pour des raisons de calcification dues à une imprégnation anormale des tissus par des sels de calcium suite à une dégénérescence des fibres de collagène. Il en résulte une rigidification du tissu de la valve et une perte de flexibilité lors du mouvement des lèvres provoquées sous l'action de la pompe cardiaque. Ces lèvres formant la valve sont en effet soumises à un mouvement continu d'ouverture et de fermeture de l'aorte à une vitesse correspondant au rythme de pulsation cardiaque, de sorte que toute rigidification du tissu les constituant entraîne irrémédiablement une usure accélérée par fatigue.

Cette usure de la valve cardiaque aura deux types de conséquences, à savoir une symphyse de valvule créant un rétrécissement aortique responsable d'un obstacle à l'éjection du ventricule gauche et une destruction des valves créant un reflux diastolique vers le ventricule gauche. Ces deux effets conduisent à une insuffisance cardiaque.

Pour réparer les insuffisances dues aux détériorations de la valve cardiaque, les premiers procédés utilisés pour construire des valves prothétiques mécaniques consistaient à les réaliser sous forme d'une bille en matériau biocompatible logée dans une cage de retenue formant simultanément un siège étanche du côté tourné vers le muscle cardiaque, ces valves étant montées à la sortie du muscle cardiaque, dans l'extrémité de l'artère comportant les sinus de reflux du flux sanguin lors de la fermeture de la valve.

Actuellement les valves mécaniques sont construites avec un ou plusieurs clapets montés sur des axes excentrés solidaires d'un de fixation sur le muscle cardiaque.

Ces valves mécaniques connues présentent une fiabilité de fonctionnement excellente, mais, cependant, elles occasionnent des turbulences qui peuvent être à l'origine d'un phénomène risquant d'entraîner des thromboses, de sorte que leur pose nécessite pour le patient ainsi équipé la consommation à vie d'un produit médicamenteux anti-coagulant.

Pour obvier à ces inconvénients il a été proposé une utilisation de bioprothèses, c'est-à-dire de prothèses réalisées à partir de tissus organiques humains (homogreffes) ou encore de prothèses porcines, c'est-à-dire de valves cardiaques porcines (US-A-5 500 015).

Les feuillets biologiques de telles prothèses sont généralement très bien supportées en greffe. Cependant, du fait même de leur constitution par des tissus organiques, elles sont sujettes au vieillissement et à la dégénérescence naturelle. Ces feuillets biologiques sont montés sur une assise qui génère un frein hémodynamique à l'éjection.

Enfin, il a également été proposé de réaliser des prothèses de valve en matière synthétique, notamment en polyuréthane ou silicone formé. Toutefois, ces valves présentent des problèmes de résistance en fatigue avec des risques de rupture au niveau des zones de flexion.

Par ailleurs, CH-A-510 433 décrit des formes destinées à la réalisation de cultures cellulaires, en vue d'effectuer une implantation ultérieure desdites cultures cellulaires. Ce document ne divulgue, cependant, aucun procédé ni moyen de réalisation de prothèse artificielle.

En outre, par WO-A-01 52 776, on connaît une prothèse de tube aortique, qui est destinée à se raccorder à une valve cardiaque. Cette prothèse n'est donc pas une prothèse de valve cardiaque proprement dite mais concerne uniquement l'environnement d'une telle valve, à savoir des sinus extérieurs qui sont fixes ou statiques.

Enfin, EP-A-0 331 345 décrit une structure textile réalisée par un tissage en trois directions, cette structure étant mise en forme par enduction. Il en résulte que la valve obtenue est un composite tissu plus polymère et présente une interface entre le tissu et le polymère, qui peut être sujette à un décollement. De plus, la structure textile bloque les fibres et empêche le glissement mutuel, le blocage des fibres les unes par rapport aux autres étant renforcé par l'adjonction de résine polymère. Il s'ensuit, que les matériaux constitutifs d'une telle valve subissent une fatigue rapide.

La présente invention a pour but de pallier ces inconvénients en proposant un procédé de réalisation d'une prothèse de valve cardiaque aortique ou mitrale et une prothèse de valve cardiaque ainsi obtenue permettant l'obtention d'une prothèse parfaitement biocompatible et d'une excellente tenue au vieillissement.

A cet effet, le procédé de réalisation d'une prothèse de valve cardiaque aortique ou mitrale est caractérisé en ce qu'il consiste essentiellement à réaliser ladite prothèse par mise en forme d'une matière textile, en particulier par mise en forme des lèvres dans une membrane textile de type choisi dans le groupe formé par les modes d'assemblage suivants : tissu, non-tissé sous forme de fibres assemblées, non-tissé obtenu par autofibrillation d'une membrane par étirage et tricot, la mise en forme des lèvres étant effectuée dans un élément tubulaire en une membrane textile, par un emboutissage concentrique de trois lèvres de surfaces identiques sur une pièce de forme reproduisant la géométrie des lèvres d'une valve cardiaque en position de fermeture de l'artère, avec découpe préalable dudit élément tubulaire sur des segments cylindriques s'étendant entre les rayons desdites lèvres, ledit élément tubulaire étant maintenu fermement au-dessus des segments faisant l'objet des découpes préalables et pouvant glisser sur la partie inférieure de la pièce de forme reproduisant la géométrie des lèvres pour former lesdites lèvres sans déformation d'allongement plastique des fibres mais avec déformation réduite de la membrane ou des mailles du tissu, à munir les bords découpés des lèvres d'un moyen de protection et de jointoiement entre lèvres en position de fermeture de la valve, puis à effectuer, éventuellement, une thermofixation de la membrane ou du tissu ainsi déformé par passage dans un four à une température légèrement supérieure à la température de transition vitreuse et, enfin, à démonter la prothèse de valve obtenue de la pièce de forme, après son refroidissement éventuel, tel que revendiqué.

L'invention a également pour objet une prothèse de valve cardiaque aortique ou mitrale, obtenue par mise en oeuvre de ce procédé, caractérisée en ce qu'elle est essentiellement constituée par une matière textile sous forme d'une membrane textile constituée par des fibres de polyester ou de polytétrafluoréthylène prises seules ou en mélange ou encore par une membrane autofibrillée par étirage de polytétrafluoréthylène et présentant une structure filamentaire et fibreuse limitant les concentrations de contrainte, ainsi qu'une rigidité de flexion faible et une rigidité en traction élevée, telle que revendiquée.

L'invention sera mieux comprise, grâce à la description ci-après, qui se rapporte à un mode de réalisation préféré, donné à titre d'exemple non limitatif, et expliqué avec référence au dessin schématique annexé dans lesquels :
la figure 1 est une vue en perspective de la prothèse conforme à l'invention ;
la figure 2 est une vue en perspective du support d'emboutissage de la prothèse suivant la figure 1 ;
la figure 3 est une vue en élévation latérale, à plus grande échelle, explicitant l'opération d'emboutissage, et
la figure 4 est une vue en plan correspondant à la figure 3 et représentant schématiquement les trois lèvres de la prothèse et les directions d'emboutissage.

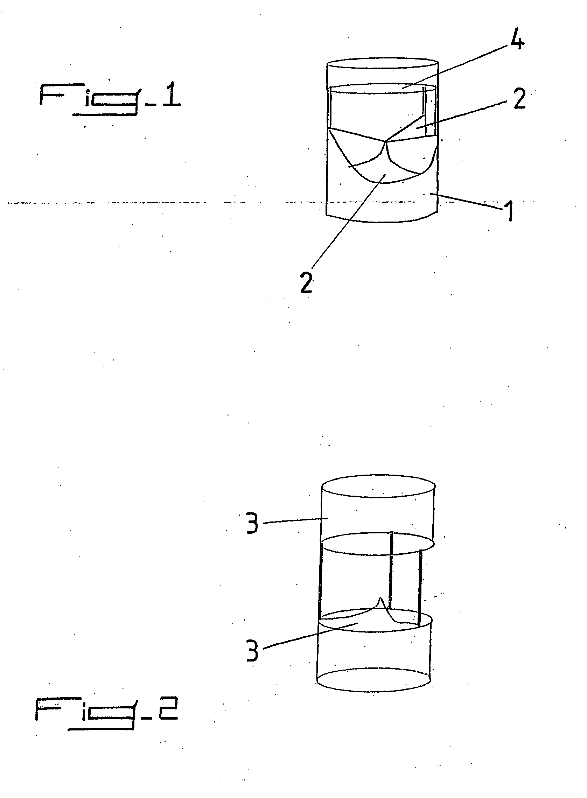

La figure 1 des dessins annexés représente une prothèse de valve cardiaque 1 essentiellement constituée par trois lèvres 2.

Cette prothèse de valve cardiaque aortique ou mitrale est avantageusement réalisée, conformément à l'invention, suivant un procédé qui consiste essentiellement à réaliser ladite prothèse par mise en forme d'une matière textile.

Selon une caractéristique de l'invention, les lèvres 2 de la prothèse de valve cardiaque 1 sont avantageusement réalisées par mise en forme d'une membrane textile. Cette membrane textile est une membrane de type choisi dans le groupe formé par les modes d'assemblage suivants : tissu, non-tissé sous forme de fibres assemblées, non-tissé obtenu par autofibrillation d'une membrane par étirage et tricot.

La mise en forme est effectuée selon une opération choisie dans le groupe suivant : emboutissage concentrique, tissage en trois dimensions, emboutissage à plat, découpe par détourage et fixation et, éventuellement, thermofixation, déformation mécanique préalable et application, au niveau des déformations, d'un fluide caloporteur, application sur une contre-forme par un effet de succion à travers ladite contre-forme et thermo-fixation par un apport d'un air ou d'un gaz chaud aspiré à travers la membrane textile dans la contre-forme, fluide caloporteur plaquant la membrane textile contre une contre-forme.

Conformément à un premier mode de réalisation de l'invention et comme le montrent plus particulièrement les figures 2 à 4 des dessins annexés, la mise en forme des lèvres 2 peut être effectuée dans un élément tubulaire 1 en une membrane textile par un emboutissage concentrique de trois lèvres 2 de surfaces identiques sur une pièce de forme 3 reproduisant la géométrie des lèvres 2 d'une valve cardiaque en position de fermeture de l'artère, avec découpe préalable dudit élément tubulaire 1 sur des segments cylindriques 4 s'étendant entre les rayons desdites lèvres 2. A cet effet, l'élément tubulaire 1 est maintenu fermement au-dessus des segments 4 faisant l'objet des découpes préalables et peut glisser sur la partie inférieure de la pièce de forme 3 reproduisant la géométrie des lèvres 2 pour former lesdites lèvres sans déformation d'allongement plastique des fibres, mais avec déformation réduite de la membrane ou des mailles du tissu. Les bords découpés des lèvres 2 sont alors pourvus d'un moyen de protection et de jointoiement entre lèvres en position de fermeture de la valve. En fonction du matériau utilisé pour la membrane textile constituant l'élément tubulaire 1, il est ensuite effectué, éventuellement, une thermofixation de ladite membrane ainsi déformée, par passage dans un four à une température légèrement supérieure à la température de transition vitreuse, puis, après refroidissement éventuel, la prothèse de valve ainsi obtenue est démontée de la pièce de forme 3.

La matière textile utilisée est avantageusement constituée par des fibres de polyester ou de polytétrafluoréthylène prises seules ou en mélange ou encore par une membrane autofibrillée par étirage de polytétrafluoréthylène.

Dans le cas d'utilisation de fibres de polyester mélangées ou non à des fibres de polytétrafluoréthylène, il est nécessaire d'effectuer une thermofixation dans un four, ce à une température de l'ordre de 120° C, la température de transition vitreuse des fibres étant de l'ordre de 80° C dans l'atmosphère pour le polyester.

Par contre, dans le cas d'utilisation d'une membrane autofibrillée par étirage de polytétrafluoréthylène, l'emboutissage successif à la découpe au niveau des segments entre les rayons permet l'obtention de lèvres 2 légèrement en forme de poches par déformation plastique permanente, sans nécessiter un quelconque traitement ultérieur de fixation.

Il en résulte l'obtention de lèvres 2 présentant une géométrie identique à celle des lèvres d'une valve humaine, permettant le respect de l'hémodynamisme.

De préférence, chaque lèvre 2 s'étend sur un segment circulaire présentant un angle d'ouverture de 120° sur le périmètre, de sorte que le bord libre de chaque poche délimitée par chaque lèvre 2 présentera une longueur, correspondant à deux fois la longueur du rayon de la lèvre, qui sera légèrement inférieure à celle définie par ledit angle d'ouverture sur le périmètre, longueur qui correspondra à deux fois le rayon multiplié par π et divisé par trois. Il en résulte que les bords des lèvres ont tendance, en coopération avec le segment circulaire délimitant chaque lèvre, à déformer lesdites lèvres en forme de poche se rabattant vers le haut en direction du sinus de l'artère, ce qui permet de garantir une des conditions de fermeture de la valve pendant la phase diastolique.

Selon une caractéristique de l'invention, la découpe préalable de l'élément tubulaire 1 sur des segments cylindriques 4 s'étendant entre les rayons desdites lèvres 2 est préférentiellement réalisée, au niveau de chaque lèvre 2, au moyen d'un dispositif de coupe, non représenté, à lame ou chauffant ou autre sectionnant l'élément tubulaire en matière textile 1 sur toute la longueur du segment avec un positionnement dudit dispositif légèrement avant le début de l'emboutissage concentrique simultané des trois lèvres 2 sur la pièce de forme 3.

Le maintien ferme de l'élément tubulaire 1 au-dessus des segments 4 pendant la découpe préalable et l'emboutissage concentrique peut être assuré, de manière connue, par une simple bride non décrite en détail. Ce maintien peut permettre d'assurer, d'une part, la création, en combinaison avec le support d'emboutissage 3, d'arêtes de coupe coopérant avec le dispositif de coupe correspondant et, d'autre part, le blocage du tissu sur tout le périmètre de la partie supérieure dudit support d'emboutissage, de sorte que lors de l'emboutissage concentrique, l'élément tubulaire en matière textile ne peut pas se déplacer du haut vers le bas en direction des poinçons d'emboutissage, alors que la partie de l'élément tubulaire en matière textile s'étendant sur la partie inférieure du support d'emboutissage 3 reproduisant la géométrie des lèvres 2 peut glisser sur cette dite partie inférieure.

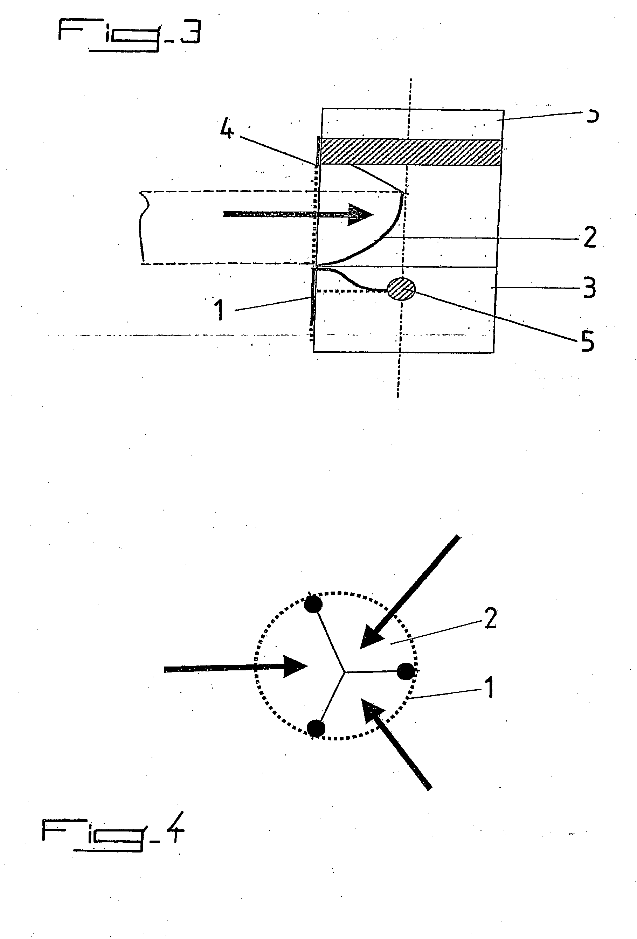

Cette notion de maintien de la matière textile sur la partie supérieure et de possibilité de glissement sur la partie inférieure est représentée plus particulièrement sur la figure 3 des dessins annexés. Il en résulte que la déformation de la matière textile au niveau des emboutissages s'effectue de manière parfaitement équilibrée sur l'ensemble des zones embouties, le remplissage des cavités de la partie inférieure du support d'emboutissage 3 s'effectuant essentiellement par un glissement de la matière textile, d'une part, entre la zone où elle est découpée, à la partie supérieure du support 3 dans chaque segment formé entre les extrémités des rayons constitutifs des lèvres 2 et, d'autre part, à partir du bas dudit support 3. Ainsi, toute possibilité de réalisation d'une zone à maille élémentaire trop élargie, donc présentant une étanchéité non équilibrée est évitée, la structure de la matière textile au niveau des lèvres 2 restant très proche de la structure de la matière textile de départ.

La structure de support d'emboutissage 3 est préférentiellement constituée par une partie inférieure cylindrique comportant une partie centrale moulée avec des formes correspondant à la forme des lèvres 2 à obtenir et par une partie supérieure cylindrique reliée à la partie inférieure par l'intermédiaire de trois tiges de maintien s'étendant entre elles à des intervalles de 120° et implantées au droit de la jonction des rayons constituant lesdites lèvres 2 avec le segment angulaire correspondant, ladite partie supérieure présentant, en direction de la partie inférieure, une surface parfaitement plane et une arête extérieure avantageusement coupante. La hauteur des tiges sera fonction de la géométrie de la poche, formée dans chaque lèvre 2, à obtenir et, en tous les cas, doit permettre une répartition optimale des déformations.

A la hauteur des tiges de maintien du support d'emboutissage, l'élément tubulaire en matière textile ne subit pas la découpe, de sorte que la pièce emboutie obtenue présente une partie inférieure munie des lèvres 2 et prolongée vers le bas par une jupe cylindrique, ladite jupe cylindrique avec les lèvres 2 étant reliée par des éléments de paroi de relativement faible largeur à un anneau cylindrique supérieur, dont la hauteur peut correspondre à la hauteur de la bride de serrage de l'élément annulaire sur le support d'emboutissage 3. La largeur de ces éléments de paroi sera sensiblement égale au diamètre ou à la largeur des tiges de maintien reliant les parties supérieure et inférieure du support d'emboutissage 3.

En outre, la hauteur, aussi bien de l'anneau cylindrique supérieur que de la jupe cylindrique inférieure peut être adaptée, en fonction des besoins par découpe d'une bande de matière textile plus ou moins importante.

De préférence, l'élément tubulaire en matière textile est bloqué ponctuellement, à trois intervalles réguliers de 120° sur la partie inférieure du support d'emboutissage 3, par l'intennédiaire de moyens de blocage 5, tels que des points de retenue (figure 3), ces moyens de blocage 5 s'étendant dans le prolongement des tiges de maintien reliant les parties supérieure et inférieure dudit support d'emboutissage 3. La prévision de ces moyens de blocage 5 permet d'assurer lors de l'opération d'emboutissage et du déplacement partiel de la matière textile sur la partie inférieure du support d'emboutissage 3, un maintien sans froissement du tube annulaire de matière textile et plus particulièrement d'éviter un froissement de ce tube au niveau des éléments de paroi reliant l'anneau tubulaire supérieur à la jupe tubulaire inférieure constituant la prothèse de valve. De même, une déformation par froissement de la jupe tubulaire inférieure est également évitée. Il en résulte que la prothèse de valve obtenue présente aussi bien une surface extérieure qu'une surface intérieure parfaitement continue.

Conformément à une autre caractéristique de l'invention, la structure fibreuse constituant l'élément tubulaire en matière textile peut être constituée, soit sous forme d'une bande longitudinale refermée sur elle-même le long d'une génératrice longitudinale par couture, soit sous forme -d'un tube continu, sans couture.

Selon une autre caractéristique de l'invention, cette structure fibreuse peut être pourvue d'un revêtement de matériau ou d'inserts de matériau améliorant son état de surface ou favorisant une réhabitation et/ou ayant des propriétés physiologiques particulières. Ainsi, la structure fibreuse pourra être pourvue, notamment, d'un revêtement ou d'inserts de substance médicamenteuse à dissolution très lente, par exemple pour éviter la formation de caillots ou encore d'une colonie de cellules endothéliales.

De préférence, la matière textile constitutive de la prothèse de valve selon l'invention est avantageusement une membrane microporeuse permettant un échange faible de fluides entre les deux faces des lèvres 2.

La mise en place sur les bords découpés des lèvres 2 d'un moyen de protection et de jointoiement entre lèvres en position de fermeture de la valve consiste avantageusement essentiellement en un traitement des parties découpées de chaque lèvre 2, à savoir des rayons de jonction au segment circulaire correspondant, par dépôt d'un matériau de recouvrement des arêtes découpées, sous forme d'un bourrelet ou analogue. La mise en place d'un tel matériau permet, d'une part, d'assurer une disposition parfaite des fils de trame et de chaîne au niveau des arêtes découpées en évitant leur effilochage et, d'autre part, de former un dispositif d'étanchéité au niveau de l'appui des bords des lèvres 2 (entre le rayon), lors de la fermeture de la valve au moment de la diastole.

Selon une variante de réalisation de l'invention, non représentée aux dessins annexés, il est également possible de réaliser une prothèse de valve cardiaque aortique ou mitrale essentiellement en effectuant la mise en forme par un tissage en trois dimensions de trois lèvres de surfaces identiques reproduisant la géométrie des lèvres d'une valve cardiaque en position de fermeture de l'artère, ce tissage étant effectué avec des fils de fibres de matière textile synthétique biocompatible.

Avec un tel mode de réalisation il est possible d'affecter chaque lèvre 2, directement lors de la fabrication, des caractéristiques mécaniques nécessaires à sa bonne tenue, tant au moment de la systole que sous pression diastolique et d'assurer simultanément une finition parfaite et optimale des bords en contact desdites lèvres.

Conformément à une autre variante de réalisation de l'invention, non représentée aux dessins annexés, il est également possible de réaliser une prothèse de valve cardiaque aortique ou mitrale en effectuant la mise en forme des lèvres 2 par emboutissage d'une membrane textile disposée à plat sur un moule correspondant, puis en effectuant la découpe par détourage desdites lèvres et la fixation et éventuellement la thermofixation de la membrane.

Conformément à une autre variante de réalisation de l'invention, non représentée aux dessins annexés, la mise en forme des lèvres 2 peut aussi être réalisée par légère déformation mécanique préalable de la zone de lisière d'une membrane textile en direction d'une contre-forme correspondante, puis par application, au niveau des déformations, d'un fluide caloporteur. Le fluide caloporteur utilisé peut être un fluide gazeux, de la vapeur, ou un fluide liquide. La réalisation des lèvres 2 directement dans la zone de lisière permet de confondre le bord des lèvres 2 à obtenir avec le bord de la membrane textile. Ainsi, après déformation, aucune partie de membrane n'est à séparer de la membrane textile au niveau des déformations des lèvres 2, de sorte que tout risque de dégradation de la membrane à ce niveau, suite à un découpage, est évité. Il en résulte que la valve obtenue est particulièrement stable dimensionnellement et mécaniquement. Par ailleurs, dans un tel cas, l'anneau supérieur de fixation peut être supprimé, de sorte que la valve obtenue présente encore une plus grande analogie avec une valve naturelle.

Il est également possible de réaliser la mise en forme des lèvres 2 par application de la zone de lisière d'une membrane textile sur une contre-forme par un effet de succion à travers ladite contre-forme, une thermo-fixation des lèvres obtenues étant réalisée par un apport d'un air ou d'un gaz chaud aspiré à travers la membrane textile dans la contre-forme.

Ainsi, la structure filamentaire et fibreuse de la membrane textile subit une déformation par mise en forme, qui favorise un déplacement libre et mieux réparti des différents points de la membrane, limitant ainsi les concentrations de contrainte.

Dans le cas d'une mise en forme avec légère déformation mécanique préalable, cette déformation mécanique peut être réalisée par l'intermédiaire d'un poinçon déplaçant les parties de membranes en direction des évidements correspondants de la contre-forme, la déformation définitive et la thermofixation étant réalisée ensuite par une circulation d'un fluide caloporteur, après retrait des poinçons ou analogues.

Dans le cas d'utilisation d'un fluide caloporteur liquide, celui-ci est projeté à une certaine pression contre la zone de lisière de la membrane textile, qui vient alors épouser la contre-forme. Dans ce mode de réalisation, il est bien entendu nécessaire d'assurer la circulation du fluide caloporteur.

Selon une autre caractéristique de l'invention, il est également possible de réaliser la mise en forme des lèvres 2 uniquement par utilisation d'un fluide caloporteur plaquant la zone de lisère de la membrane textile contre une contre-forme et présentant une température légèrement supérieure à celle de transition vitreuse du textile utilisé.

Enfin, selon une autre caractéristique de l'invention, dans le cas d'une réalisation des lèvres 2 directement dans la zone de lisière d'une membrane textile, le bord libre de cette zone comporte avantageusement une lisière tissée ou tricotée évitant un effilochage dudit bord. Une telle réalisation est particulièrement importante, du fait qu'elle permet de supprimer tout traitement ultérieur ou antérieur du bord, qui pourrait avoir pour conséquence une modification des caractéristiques mécaniques de la membrane au niveau du bord, ou une modification de l'épaisseur de la membrane à ce niveau.

Grâce à l'invention, il est possible de réaliser une prothèse de valve cardiaque aortique ou mitrale pouvant très facilement être implantée, par suture ou par l'intermédiaire d'éléments mécaniques expansibles restant à demeure, ou autre, et présentant une structure à rigidité de flexion faible, favorable à la contrainte de flexion en fatigue et associée à une rigidité en traction élevée, favorable à la contrainte de membrane.

La structure de la membrane textile utilisée permet de répondre de manière optimale aux sollicitations privilégiées de la valve originelle, à savoir radiale et circonférentielle, avec un couplage entre les deux. La compliance de la valve en matière textile respecte donc celle de la valve réelle.

En outre, les différents procédés de fabrication permettent d'obtenir une valve présentant une géométrie identique à celle de la valve humaine, respectant donc l'hémodynamisme. En particulier, la quasi-égalité de longueur entre la somme des rayons formant les deux bords libres de chaque lèvre 2 et le segment circulaire correspondant permet de garantir une des conditions de fermeture de la valve pendant la phase diastolique.

Par ailleurs, la prévision de la jupe circulaire inférieure, ainsi que, éventuellement, de l'anneau tubulaire supérieur permettent, soit d'éviter éventuellement l'utilisation d'un châssis, de sorte que l'écoulement n'est pas perturbé par suture directe des deux éléments cylindriques sur l'artère avec une orientation optimale par rapport à l'écoulement, soit d'envisager une implantation par utilisation d'un dispositif de serrage et de maintien en place par expansion de type stent, ou par l'intermédiaire d'un dispositif de suture endoscopique, voire pour une implantation sans perturbation de l'écoulement sanguin.

Bien entendu, l'invention n'est pas limitée au mode de réalisation décrit et représenté aux dessins annexés. Des modifications restent possibles, notamment du point de vue de la constitution des divers éléments ou par substitution d'équivalents techniques, sans sortir pour autant du domaine de protection de l'invention.

## Revendications

1. Procédé de réalisation d'une prothèse de valve cardiaque aortique ou mitrale **caractérisé en ce qu'**il consiste essentiellement à réaliser ladite prothèse par mise en forme d'une matière textile, en particulier par mise en forme des lèvres (2) dans une membrane textile de type choisi dans le groupe formé par les modes d'assemblage suivants : tissu, non-tissé sous forme de fibres assemblées, non-tissé obtenu par autofibrillation d'une membrane par étirage et tricot, la mise en forme des lèvres (2) étant effectuée soit :
- dans un élément tubulaire (1) en une membrane textile, par un emboutissage concentrique de trois lèvres (2) de surfaces identiques sur une pièce de forme (3) reproduisant la géométrie des lèvres d'une valve cardiaque en position de fermeture de l'artère, avec découpe préalable dudit élément tubulaire (1) sur des segments cylindriques (4) s'étendant entre les rayons desdites lèvres (2), ledit élément tubulaire (1) étant maintenu fermement au-dessus des segments (4) faisant l'objet des découpes préalables et pouvant glisser sur la partie inférieure de la pièce de forme (3) reproduisant la géométrie des lèvres (2) pour former lesdites lèvres (2) sans déformation d'allongement plastique des fibres mais avec déformation réduite de la membrane ou des mailles du tissu, à munir les bords découpés des lèvres (2) d'un moyen de protection et de jointoiement entre lèvres en position de fermeture de la valve, puis à effectuer, éventuellement, une thermofixation de la membrane ou du tissu ainsi déformé par passage dans un four à une température légèrement supérieure à la température de transition vitreuse et, enfin, à démonter la prothèse de valve obtenue de la pièce de forme (3), après son refroidissement éventuel,
- par un tissage en trois dimensions de trois lèvres (2) de surfaces identiques reproduisant la géométrie des lèvres (2) d'une valve cardiaque en position de fermeture de l'artère, ce tissage étant effectué avec des fils de fibres de matière textile synthétique biocompatible,
- par un emboutissage d'une membrane textile disposée à plat sur un moule correspondant, suivie de la découpe par détourage desdites lèvres (2) et la fixation et, éventuellement, la thermofixation de la membrane,
- par une mise en forme des lèvres (2) par légère déformation mécanique préalable de la zone de lisière d'une membrane textile en direction d'une contre-forme correspondante, puis par application, au niveau des déformations, d'un fluide caloporteur,
- par une mise en forme par application de la zone de lisière d'une membrane textile sur une contre-forme par un effet de succion à travers ladite contre-forme, une thermo-fixation des lèvres (2) obtenues étant réalisée par un apport d'un air ou d'un gaz chaud aspiré à travers la membrane textile dans la contre-forme, ou
- par une opération de mise en forme réalisée uniquement par utilisation d'un fluide caloporteur plaquant la zone de lisière de la membrane textile contre une contre-forme et présentant une température supérieure à celle de transition vitreuse du textile utilisé.

2. Procédé, suivant la revendication 1, **caractérisé en ce que** la découpe préalable de l'élément tubulaire (1) sur des segments cylindriques (4) s'étendant entre les rayons desdites lèvres (2) est réalisée, au niveau de chaque lèvre (2), au moyen d'un dispositif de coupe sectionnant l'élément tubulaire en matière textile (1) sur toute la longueur du segment avec un positionnement dudit dispositif légèrement avant le début de l'emboutissage concentrique simultané des trois lèvres (2) sur la pièce de forme (3).

3. Procédé, suivant la revendication 1, **caractérisé en ce que** l'élément tubulaire (1) en matière textile est bloqué ponctuellement, à trois intervalles réguliers de 120° sur la partie inférieure du support d'emboutissage (3) par l'intermédiaire de moyens de blocage (5), tels que des points de retenue, ces moyens de blocage (5) s'étendant dans le prolongement des tiges de maintien reliant les parties supérieure et inférieure dudit support d'emboutissage (3).

4. Procédé, suivant l'une quelconque des revendications 1 à 3, **caractérisé en ce qu'**à la hauteur des tiges de maintien du support d'emboutissage, l'élément tubulaire (1) en matière textile ne subit pas la découpe, de sorte que la pièce emboutie obtenue présente une partie inférieure munie des lèvres (2) et prolongée vers le bas par une jupe cylindrique, ladite jupe cylindrique avec les lèvres (2) étant reliée par des éléments de paroi de relativement faible largeur à un anneau cylindrique supérieur, la largeur de ces éléments de paroi étant sensiblement égale au diamètre ou à la largeur des tiges de maintien reliant les parties supérieure et inférieure du support d'emboutissage (3).

5. Procédé, suivant la revendication 1, **caractérisé en ce que** la structure fibreuse constituant l'élément tubulaire (1) en matière textile est constituée, soit sous forme d'une bande longitudinale refermée sur elle-même le long d'une génératrice longitudinale par couture, soit sous forme d'un tube continu, sans couture.

6. Procédé, suivant la revendication 1, **caractérisé en ce que** la mise en place sur les bords découpés des lèvres (2) d'un moyen de protection et de jointoiement entre lèvres en position de fermeture de la valve consiste essentiellement en un traitement des parties découplées de chaque lèvre (2), à savoir les rayons de jonction au segment circulaire correspondant, par dépôt d'un matériau de recouvrement des arêtes découpées, sous forme d'un bourrelet ou analogue.

7. Procédé, suivant la revendication 1, **caractérisé en ce que** la légère déformation mécanique préalable est réalisée par l'intermédiaire d'un poinçon déplaçant les parties de membranes en direction des évidements correspondants de la contre-forme, la déformation définitive et la thermofixation étant réalisée ensuite par une circulation d'un fluide caloporteur, après retrait des poinçons.

8. Dispositif pour la mise en oeuvre du procédé suivant l'une quelconque des revendications 1 à 7, **caractérisé en ce que** la structure de support d'emboutissage (3) est constituée par une partie inférieure cylindrique comportant une partie centrale moulée avec des formes correspondant à la forme des lèvres (2) à obtenir et par une partie supérieure cylindrique reliée à la partie inférieure par l'intermédiaire de trois tiges de maintien s'étendant entre elles à des intervalles de 120 ° et implantées au droit de la jonction des rayons constituant lesdites lèvres (2) avec le segment angulaire correspondant, ladite partie supérieure présentant, en direction de la partie inférieure, une surface parfaitement plane et une arête extérieure coupante,

9. Dispositif, suivant la revendication 8, **caractérisé en ce que** le support d'emboutissage (3) est pourvu de moyens de blocage (5), tels que des points de retenue, ces moyens de blocage (5) s'étendant dans le prolongement des tiges de maintien reliant les parties supérieure et inférieure dudit support d'emboutissage (3).

10. Prothèse de valve cardiaque aortique ou mitrale, obtenue par mise en oeuvre du procédé suivant l'une quelconque des revendications 1 à 7, essentiellement constituée par une matière textile sous forme d'une membrane textile constituée par des fibres de polyester ou de polytétrafluoréthylène prises seules ou en mélange ou encore par une membrane autofibrillée par étirage de polytétrafluoréthylène et présentant une structure filamentaire et fibreuse limitant les concentrations de contrainte, ainsi qu'une rigidité de flexion faible et une rigidité on traction élevée, **caractérisée en ce que** dans les cas d'une réalisation des lèvres (2) directement dans la zone de lisière d'une membrane textile, le bord libre de cette zone comporte une lisière tissée ou tricotée évitant un effilochage dudit bord.

11. Prothèse, suivant la revendication 10, **caractérisé en ce que** la matière textile est pourvue d'un revêtement de matériau ou d'inserts de matériau améliorant son état de surface ou favorisant une réhabitation et/ou ayant des propriétés physiologiques particulières.

12. Prothèse, suivant la revendication 11, **caractérisé en ce que** le matériau textile est muni d'un revêtement ou d'inserts de substance médicamenteuse à dissolution très lente, par exemple pour éviter la formation de caillots ou encore d'une colonie de cellules endothéliales.

13. Prothèse, suivant l'une quelconque des revendications 10 à 12, **caractérisé en ce que** la matière textile la constituant est une membrane microporeuse.

14. Prothèse, obtenue par mise en oeuvre du procédé suivant l'une quelconque des revendications 1 à 7, **caractérisé en ce que** chaque lèvre (2) s'étend sur un segment circulaire présentant un angle d'ouverture de 120° sur le périmètre, de sorte que le bord libre de chaque poche délimitée par chaque lèvre (2) présente une longueur, correspondant à deux fois la longueur du rayon de la lèvre, qui est légèrement inférieure à celle définie par ledit angle d'ouverture sur le périmètre, longueur qui correspond à deux fois le rayon multiplié par π et divisé par trois.

## Patentansprüche

1. Verfahren zur Herstellung einer Aorten- oder Mitralklappenprothese, **dadurch gekennzeichnet, dass** es im Wesentlichen darin besteht, die genannte Prothese durch Formen eines Textilwerkstoffes, insbesondere durch Formen der Ventillappen (2) aus einer Textilmembran einer Art, die aus der Gruppe der folgenden Verbundarten ausgewählt ist: Gewebe, Faservlies in Form verbundener Fasern, durch Zerfasern einer Membran durch Strecken gefertigtes Faservlies und Gestrick, wobei die Formung der Ventillappen (2) in folgender Weise erfolgt:
- entweder in einem rohrförmigen Teil (1) aus einer Textilmembran durch ein konzentrisches Eindrücken von drei Ventillappen (2) identischer Oberfläche auf einem Formstück (3), das die Geometrie der Ventillappen einer Herzklappe in der Arterienverschlussstellung reproduziert, mit vorherigem Zuschnitt des genannten rohrförmigen Teils (1) in Zylinderabschnitte (4), die sich zwischen den Radien der genannten Ventillappen (2) erstrecken, wobei das genannte rohrförmige Teil (1) oberhalb der Abschnitte (4), die Gegenstand der vorherigen Zuschnitte sind, festgehalten wird und auf den unteren Teil des Formstückes (3), das die Geometrie der Ventillappen (2) reproduziert, gleiten kann, um die genannten Ventillappen (2) ohne Verformung durch plastische Streckung der Fasern, sondern durch geringfügige Verformung der Membran oder der Maschen des Gewebes zu formen, die ausgeschnittenen Kanten der Ventillappen (2) mit einem Mittel zum Schutz und zur Verfugung der Ventillappen in der Verschlussstellung des Ventils zu versehen, und dann gegebenenfalls eine Thermofixierung der Membran oder des so verformten Stoffes in einem Ofen bei geringfügig höherer Temperatur, als der Glasübergangstemperatur, vorzunehmen, und schließlich die erhaltene Klappenprothese vom Formstück (3) abzunehmen, gegebenenfalls nach dem Abkühlen,
- durch dreidimensionales Weben dreier Ventillappen (2) mit identischen Oberflächen, die die Geometrie der Ventillappen (2) einer Herzklappe in der Verschlussstellung der Arterie reproduzieren, wobei dieses Weben mit Fasergarnen aus biokompatiblen, synthetischen Textilwerkstoffen erfolgt, oder
- durch Eindrücken einer Textilmembran, die flach auf einer entsprechenden Form angeordnet ist, gefolgt vom Zuschnitt durch Ausschneiden der genannten Ventillappen (2) und der Verbindung und gegebenenfalls dem Thermofixieren der Membran,
- durch Formen der Ventillappen (2) durch geringfügige mechanische Vorverformung des Randbereichs einer Textilmembran in Richtung auf eine entsprechende Gegenform und dann Anwendung eines Heizfluids auf der Höhe der Verformungen.
- durch eine Formgebung durch Andrücken des Randbereichs einer Textilmembran an eine Gegenform durch Ansaugen durch die genannte Gegenform hindurch, wobei ein Thermofixieren der erhaltenen Ventillappen (2) durch Anwendung heißer Luft oder heißen Gases durch die Textilmembran hindurch in die Gegenform erfolgt, oder
- durch eine Formgebung, die ausschließlich durch Verwendung eines Heizfluids erfolgt, das den Randbereich der Textilmembran an eine Gegenform anlegt und eine Temperatur aufweist, die die Glasübergangstemperatur des verwendeten Textils überschreitet.

2. Verfahren nach Patentanspruch 1, **dadurch gekennzeichnet, dass** der vorangehende Zuschnitt des rohrförmigen Teils (1) in Zylinderabschnitte (4), die zwischen den Radien der genannten Ventillappen (2) verlaufen, im Bereich jedes Ventillappens (2) mit Hilfe einer Schneidvorrichtung erfolgt, die das rohrförmige Teil aus Textilwerkstoff (1) über die gesamte Länge des Abschnitts unter Anordnung der genannten Vorrichtung geringfügig vor dem Beginn des gleichzeitigen konzentrischen Eindrückens der drei Ventillappen (2) auf dem Formstück (3), auftrennt.

3. Verfahren nach Patentanspruch 1, **dadurch gekennzeichnet, dass** das rohrförmige Teil (1) aus Textilwerkstoff punktuell in drei regelmäßigen Intervallen von 120° auf dem unteren Teil des Eindrückträgers (3) mit Hilfe von Arretiermitteln (5), wie etwa Rückhaltepunkten, festgesetzt wird, wobei diese Arretiermittel (5) in der Verlängerung von Haltestäben liegen, die den oberen Teil und den unteren Teil des genannten Eindrückträgers (3) verbinden.

4. Verfahren nach einem der Patentansprüche 1 bis 3, **dadurch gekennzeichnet, dass** das rohrförmige Teil (1) aus Textilwerkstoff im Bereich der Haltestäbe des Eindrückträgers keinen Zuschnitt erfährt, derart, dass das gefertigte eingedrückte Teil einen unteren Abschnitt aufweist, der mit Ventillappen (2) versehen ist, und sich nach unten in einer zylindrischen Schürze fortsetzt, wobei die genannte zylindrische Schürze mit den Ventillappen (2) durch Wandteile relativ geringer Breite mit einem oberen zylindrischen Ring verbunden ist, wobei die Breite dieser Wandteile im Wesentlichen dem Durchmesser oder der Breite der Haltestäbe gleich ist, die den oberen und den unteren Teil des Eindrückträgers (3) verbinden.

5. Verfahren nach Patentanspruch 1, **dadurch gekennzeichnet, dass** die Faserstruktur, die das rohrförmige Teil (1) aus Textilwerkstoff bildet, entweder in der Form eines langgestreckten Bandes, das durch Nähen längs einer Mantellinie in sich geschlossen wird, gefertigt wurde, oder in der Form eines nahtlosen Rohres.

6. Verfahren nach Patentanspruch 1, **dadurch gekennzeichnet, dass** das Anordnen auf den Schnitträndern der Ventillappen (2) eines Mittels zum Schutz und zum Verfugen der Ventillappen in der Verschlussstellung der Klappe im Wesentlichen in einer Behandlung der zugeschnittenen Teile jedes Ventillappens (2) d. h. der Radien des Zusammenfügens mit dem entsprechenden Kreissegment, durch Auftragen eines Werkstoffes zur Beschichtung der Schnittkanten in Form eines Wulstes oder Ähnlichem besteht.

7. Verfahren nach Patentanspruch 1, **dadurch gekennzeichnet, dass** die geringfügige mechanische Vorverformung mit Hilfe eines Stempels erfolgt, der die Membranteile in Richtung der entsprechenden Aussparungen der Gegenform bewegt, wobei die endgültige Verformung und die Thermofixierung anschließend, nach einem Zurückziehen des Stempels durch die Strömung eines Heizfluids erfolgt.

8. Vorrichtung zur Durchführung des Verfahrens nach irgendeinem der Patentansprüche 1 bis 7, **dadurch gekennzeichnet, dass** der Eindrückträger (3) aus einem zylindrischen Unterteil besteht, der einen mit den Formen der zu fertigenden Ventillappen (2) geformten zentralen Teil aufweist, und aus einem zylindrischen Oberteil, das mit Hilfe dreier Haltestäbe, die sich in Abständen von 120° zueinander befinden und rechtwinklig zur Verbindung der Radien, die die genannten Ventillappen (2) bilden, mit dem entsprechenden Winkelabschnitt eingesetzt sind, mit dem Unterteil verbunden ist, wobei das genannte Oberteil in Richtung des Unterteils eine vollkommen ebene Fläche und eine scharfe Außenkante aufweist.

9. Vorrichtung nach Patentanspruch 8, **dadurch gekennzeichnet, dass** der Eindrückträger (3) mit Arretiermitteln (5), wie etwa Haltepunkten, versehen ist, wobei diese Arretiermittel (5) in der Verlängerung der Haltestäbe liegen, die den oberen und den unteren Teil des genannten Eindrückträgers (3) verbinden.

10. Aorten- oder Mitralklappenprothese, hergestellt durch das Durchführen des Verfahrens nach einem der Patentansprüche 1 bis 7, im Wesentlichen bestehend aus einem Textilwerkstoff in Form einer Textilmembran, die aus Polyester- oder Polytetrafluorethylenfasern, einzeln oder gemischt, besteht oder auch aus einer durch Strecken zerfaserten Membran aus Polytetrafluorethylen, und die eine Faserstruktur aufweist, die die Belastungskonzentrationen begrenzt, sowie eine geringe Biegefestigkeit und eine hohe Zugfestigkeit, **dadurch gekennzeichnet, dass** im Fall der Herstellung der Ventillappen (2) direkt im Randbereich einer Textilmembran die freie Kante dieses Bereichs einen gewebten oder gestrickten Rand aufweist, der ein Ausfransen des genannten Randes vermeidet.

11. Prothese nach Patentanspruch 10, **dadurch gekennzeichnet, dass** der Textilwerkstoff mit einer Beschichtung oder mit Einsätzen aus einem Werkstoff versehen ist, der seine Oberflächeneigenschaften verbessert oder eine Rehabilitation fördert und/oder besondere physiologische Eigenschaften hat.

12. Prothese nach Patentanspruch 11, **dadurch gekennzeichnet, dass** der Textilwerkstoff mit einer Beschichtung oder Einsätzen aus sich sehr langsam lösenden Medikamenten versehen ist, beispielsweise um die Bildung von Blutklümpchen oder auch einer Kolonie von Endothelzellen zu vermeiden.

13. Prothese nach irgendeinem der Patentansprüche 10 bis 12, **dadurch gekennzeichnet, dass** der Textilwerkstoff, aus dem sie besteht, eine mikroporöse Membran ist.

14. Prothese, hergestellt durch das Durchführen des Verfahrens nach irgendeinem der Patentansprüche 1 bis 7, **dadurch gekennzeichnet, dass** sich jeder Ventillappen (2) über ein Kreissegment erstreckt, das einen Öffnungswinkel von 120° auf dem Umkreis aufweist, derart, dass der freie Rand jeder Tasche, die durch jeden Ventillappen (2) begrenzt wird, eine Länge aufweist, die das Doppelte der Länge des Radius des Ventillappens beträgt, die geringfügig kleiner ist, als die durch den genannten Öffnungswinkel auf dem Umkreis definierte Länge, die dem doppelten Radius, multipliziert mit π und geteilt durch drei, entspricht.

## Claims

1. Method for the production of an aortic or mitral cardiac valve prosthesis, **characterised in that** it consists essentially in making said prosthesis by shaping a textile material, in particular by shaping flaps (2) in a textile membrane of the type selected from a group formed by the following assembly modes: woven, non-woven in the form of assembled fibres, non-woven obtained by the autofibrillation of a membrane by drawing and knitting, the shaping of the flaps (2) being carried out either:
- in a tubular element (1) made from a textile membrane, by a concentric shaping of three flaps (2) with identical surfaces on a shaping member (3) reproducing the geometry of the flaps of a cardiac valve in the closed position of the artery, with prior cutting of said tubular element (1) into cylindrical segments (4) extending between the radii of said flaps (2), said tubular element (1) being held firmly above segments (4) forming the object of preliminary cut-outs and being adapted to slide over the lower portion of the shaping member (3) reproducing the geometry of the flaps (2) to form said flaps (2) without plastic elongation deformation of the fibres but with reduced deformation of the membrane or the meshes of the fabric, in providing the cut edges of the flaps (2) with a protection and junction means between flaps in the closed position of the valve, then in carrying out, if necessary, the thermofixing of the membrane or fabric thus deformed by passage through an oven at a temperature slightly greater than the vitreous transition temperature and finally in removing the valve prosthesis obtained from the shaping member (3), after possible cooling,
- by weaving in three dimensions three flaps (2) with identical surfaces reproducing the geometry of the flaps (2) of a cardiac valve in the position of closure of the artery, this weaving being carried out with filaments of fibres of biocompatible synthetic textile material,
- by shaping a textile member disposed at on a corresponding mould, followed by cutting by clipping said flaps (2) and fixing and, possibly, thermofixing the membrane.
- by forming the flaps (2) by slight preliminary mechanical deformation of the edge zone of a textile membrane in the direction of a corresponding counter-form, then by application in line with the deformation of a heat-transferring fluid,
- by shaping by application of the edge zone of a textile membrane against a counter-form by suction through said counter-form, a thermofixation of the obtained flaps (2) being effected by a supply of hot air or hot gas drawn through the textile membrane into the counter-form or
- by a shaping operation performed exclusively by using a heat-transferring fluid pressing the edge zone of the textile membrane against a counter-form and having a temperature greater than the vitreous transformation temperature of the textile used.

2. Method according to claim 1, **characterised in that** the preliminary cutting of the tubular element (1) into cylindrical segments (4) extending between the radii of said flaps (2) is carried out, for each flap (2), by means of a cutting device sectioning the tubular element of textile material (1) over the whole length of the segment positioning the said device slightly in front of the beginning of the simultaneous concentric shaping of the three flaps (2) on the shaping member (3).

3. Method according to claim 1, **characterised in that** the tubular element (1) made of textile material is blocked at selective points, at three regular intervals of 120° on the lower portion of the shaping support (3) by means of blocking means (5) such as retention points, said blocking means (5) extending in extension of the retaining rods connecting the upper and lower portions of said shaping support (3).

4. Method according to any one of claims 1 to 3, **characterised in that** at the height of the retaining rods of the shaping support, the tubular element (1) of textile material is not subjected to cutting, such that the shaped piece obtained has a lower portion provided with flaps (2) and extended downwards by a cylindrical skirt, said cylindrical skirt with the flaps (2) being connected by wall elements of relatively small width to an upper cylindrical ring, the width of these wall elements being substantially equal to the diameter or to the width of the retaining rods connecting the upper and lower portions of the shaping support (3).

5. Method according to claim 1, **characterised in that** the fibrous structure forming the tubular element (1) of textile material is made, either in the form of a longitudinal strip closed on itself along a longitudinal generatrix by stitching or in the form of a continuous tube without stitching.

6. Method according to claim 1, **characterised in that** the arrangement on the cut edges of the flaps (2) of a protection and joining means between the flaps in the closed position of the valve consists essentially of treating the cut portions of each flap (2), namely the junction radii to the corresponding circular segment, by depositing a covering material for the cut edges, in the form of a pad or the like.

7. Method according to claim 1, **characterised in that** the slight preliminary mechanical deformation is performed by means of a punch moving the membrane portions in the direction of corresponding recesses of the counter-form, the final shaping and thermofixing being then carried out by circulation of a heat-transferring fluid, after the withdrawal of the punches.

8. Device for implementing the method according to any one of claims 1 to 7, **characterised in that** the structure of the shaping support (3) is formed by a lower cylindrical portion comprising a central moulded portion with forms corresponding to the shape of the flaps (2) to be obtained and by an upper cylindrical portion connected to the lower portion by means of three retaining rods extending between them at intervals of 120° and implanted in line with the junction of the radii constituting said flaps (2) with the corresponding angular segment, said upper portion having, in the direction of the lower portion, a perfectly flat surface and an external cutting edge.

9. Device according to claim 8, **characterised in that** the shaping support (3) is provided with blocking means (5), such as retaining points, said blocking means (5) extending in extension of the retaining rods connecting the upper and lower portions of said shaping support (3).

10. Aortic or mitral cardiac valve prosthesis, obtained by implementing the method according to any one of claims 1 to 7, essentially formed by a textile material in the form of a textile membrane formed by polyester or polytetrafluoroethylene fibres alone or in a mixture or even by a membrane autofibrillated by drawing polytetrafluoroethylene and having a filament and fibrous structure limiting the stress concentrations as well as low flexural stiffness and a high tensile rigidity, **characterised in that** in the case of producing flaps (2) directly in the edge zone of a textile membrane, the free edge of this zone comprises a woven or knit edge avoiding the fraying of said edge.

11. Prosthesis according to claim 10, **characterised in that** the textile material is provided with a coating of material or inserts of material improving its surface condition or promoting rehabilitation and/or having particular physiological properties.

12. Prosthesis according to claim 11, **characterised in that** the textile material is provided with a coating or inserts made of a very slowly dissolving pharmaceutical substance, for example to avoid the formation of clots or even a colony of endothelial cells.

13. Prosthesis according to any one of claims 10 to 12, **characterised in that** the textile material it is made of is a microporous membrane.

14. Prosthesis obtained by implementing the method according to any one of claims 1 to 7, **characterised in that** each flap (2) extends over a circular segment having an opening angle of 120° over its periphery, such that the free edge of each pocket delimited by each flap (2) has a length corresponding to twice the length of the radius of the flap, which is slightly less than that defined by said opening angle over the periphery, the length corresponding to twice the radius multiplied by π and divided by three.
